# FASCICULE DE BREVET EUROPEEN

(11) **EP 0 968 709 B1**
(45) Date de publication et mention de la délivrance du brevet: **26.11.2003**
(21) Numéro de dépôt: 99401508.9
(22) Date de dépôt: 17.06.1999
(51) Int. Cl.: A61K 7/48, A61K 7/06

(54) **Composition cosmetique exploitant des effets antiradicalaires synergiques**
Kosmetische Zusammensetzung mit antiradikalischer synergistischer Wirkung
Cosmetic composition having antiradical synergistic effect

(30) Priorité: 03.07.1998 FR 9808720
(43) Date de publication de la demande: 05.01.2000
(73) Titulaire: Lanatech Laboratoire Nature et Technique, 75009 Paris (FR)
(72) Inventeur: Fristsch, Marie-Claire, 75009 Paris (FR); Vacher, Anne-Marie, 78150 Le Chesnay (FR)
(74) Mandataire: de Saint-Palais, Arnaud Marie

(56) Documents cités:
- EP-A- 0 317 453
- WO-A-97/42928
- WO-A-98/20851
- WO-A-98/30200
- WO-A-98/55082
- FR-A- 2 096 902
- FR-A- 2 618 071
- FR-A- 2 753 374
- DATABASE WPI Week 9311 Derwent Publications Ltd., London, GB; AN 93-088593 XP002103006 "External prepn. to prevent skin ageing - contg. extract of Ginkgo biloba L and superoxidedismutase and having high prophylactic effect" & JP 05 032556 A (KOSE), 9 février 1993 (1993-02-09)

## Description

La présente invention concerne un perfectionnement à la composition pour la prévention du vieillissement cutané faisant l'objet du brevet PCT/FR/97/02017, déposé le 10 novembre 1997 nom de la Demanderesse et publié sous le numéro WO 98/20851.

Elle s'applique d'une façon analogue aussi bien au vieillissement naturel qu'aux phénomènes de vieillissement accidentels de la peau dus aux multiples agressions que subit quotidiennement la peau, en particulier, celles dues aux rayonnements solaires.

D'une manière générale, on sait que les radicaux libres (particules agressives possédant un électron libre) sont les agents principaux incriminés dans les altérations de la peau dues, par exemple, soit à des expositions prolongées aux radiations solaires et donc aux rayonnements ultraviolets inclus dans ces radiations, ou même à des réactions métaboliques et enzymatiques qui se produisent dans l'organisme.

L'attaque par voie radicalaire initie des réactions en chaîne qui ne s'arrêtent que lorsque deux radicaux libres s'inactivent mutuellement.

La peroxydation lipidique (lipoperoxydation) est un cas typique de réaction en chaîne induite par voie radicalaire.

L'oxydation des lipides membranaires aboutit à la formation de lipoperoxydes, qui se décomposent en différents produits de fragmentation dont certains sont très agressifs.

L'un des produits de fragmentation le plus important et le plus agressif est un aldhéhyde, le malone-dialdéhyde (MDA) qui manifeste une toxicité redoutable en pontant transversalement les protéines, les lipides intracellulaires et l'ADN.

Il apparaît donc que les radicaux libres et la cascade de réactions en chaîne qu'ils provoquent au sein de l'organisme jouent un rôle très important dans le processus de vieillissement cutané.

Dans le but de lutter contre cette action radicalaire, la Demanderesse propose une composition utilisant les propriétés du chrysanthellum indicum qui, selon le brevet FR précité :
- présente de très bonnes propriétés antiradicalaires, de manière à obtenir une action anti-âge aussi bien pour les vieillissements naturels ou accidentels ;
- assure une protection efficace contre les réactions aboutissant à la production de radicaux OH° ou de radicaux de type oxoferryl ;
- réduise la chute du contenu en ATP des kératinocytes après irradiation, de manière à obtenir un effet réparateur.

Jusque là, comme l'attestent de nombreuses publications scientifiques, les recherches effectuées sur le chrysanthellum indicum avaient permis de mettre en évidence les effets cliniques dans des pathologies essentiellement circulatoires et digestives (domaine exclusivement médical).

Seul le brevet FR No 87 10502 publié sous le numéro FR 2 618 071 avait proposé une composition cosmétique comprenant un extrait de chrysanthellum renfermant de 1 % à 10 % d'extrait de chrysanthellum pour des applications telles que des shampooings et des lotions capillaires, des émulsions dermiques, des laits corporels, des rouges à lèvres, voire même des compositions cosmétiques sous forme d'aérosols.

Outre le fait que ce document n'évoque pas la prévention du vieillissement cutané, les concentrations qu'il préconise conduisent à des produits totalement inappropriés et même incompatibles avec un usage cosmétique normal. En effet, à ces concentrations, les émulsions à usage topique présentent notamment :
· Une coloration marron foncé qui provoque la teinture jaune moutarde intense de la peau à traiter ainsi que celle des doigts ayant servi à effectuer l'application. La forte intensité de cette teinture s'atténue au rinçage mais laisse subsister, après lavage, une coloration jaune foncé.
· Une mauvaise stabilité dans le temps : on constate, dès 24 heures, une concentration de l'eau dans le fond du récipient et un important relargage de l'huile en surface.
· Une forte odeur d'extrait végétal.

L'invention a donc plus particulièrement pour but une composition à base de chrysanthellum indicum dans laquelle l'effet antiradicalaire se trouve renforcé, sans avoir à augmenter la teneur en chrysanthellum indicum qui doit demeurer à un niveau suffisamment bas pour éviter les inconvénients précédemment évoqués.

Elle propose, à cet effet, d'utiliser des effets antiradicalaires synergiques, c'est-à-dire obtenus par l'association au chrysanthellum d'une ou plusieurs molécules.

Or, on sait qu'une telle association peut conduire :
a) soit à une activité moins forte que l'addition de l'activité de chacun des produits, en raison d'un effet antagoniste (un produit réduisant l'activité de l'autre) ou d'un effet de saturation (l'effet maximal atteint ne pouvant pas être dépassé) ou même d'un effet néfaste (effet antagoniste prédominant) ;
b) soit à une activité égale à la somme des activités de chacun des produits testés séparément ;
c) soit à une activité supérieure à la somme des activités des produits testés séparément (effet synergique).

Dans la pratique, autant il est concevable qu'il puisse exister un seuil d'activité que l'on ne peut pas dépasser, autant il est peu plausible d'espérer obtenir des effets additionnels même partiels. La mise en évidence d'effets synergiques est, quant à elle, très rare.

En ce qui concerne le chrysanthellum indicum, aucun effet antiradicalaire synergique l'impliquant n'était connu jusqu'ici et rien ne laissait présager qu'il serait possible de mettre en évidence un effet synergique entre le chrysanthellum et d'autres molécules, ni même que ce type d'association permettrait d'améliorer des performances déjà excellentes.

Or, les études conduites par la Demanderesse on permis de révéler un tel effet synergique.

Ces études, réalisées in vitro, avaient pour but :
. d'une part, de mesurer les effets de quatre composés :
   - extrait de chrysanthellum indicum,
   - extrait de thé vert,
   - extrait de Ginkgo biloba,
   - acétate de DL-alpha tocophérol,
      vis-à-vis de la peroxydation de l'acide linoléique par les radicaux OH° et peroxyferryl,
. d'autre part, de rechercher une éventuelle potentialisation entre le chrysanthellum et l'un des autres composés.

A cet effet, chacun des composés a été soumis au traitement d'initiation de la peroxydation lipidique suivant :

La peroxydation d'une émulsion d'acide linoléique a été induite par des radicaux du type : hydroxyle OH° et oxyferryl. L'acide linoléique (10 mg) a été émulsifié par 0,2 % de 3 - [(3 cholimadopropyl) - diméthylammonio]- 1 -propane-sulfonate (CHAPS), dans un tampon phosphate pH 7,3 (50 ml).

Les radicaux OH° sont produits à l'aide d'une source d'irradiation gamma. Dans ces conditions, l'irradiation est réalisée à 20°C pendant 18 heures (Source ¹³¹ Cs, dose totale : 180 grays/18000 rads).

Les radicaux oxyferryls sont générés par addition de 10 µl d'hémoglobine dans une émulsion d'acide linoléique (2 ml), pendant 3 heures (incubation à 40°C).

Ces essais ont été réalisés dans des flacons de 10 ml étanches en présence ou en absence de deux antioxydants témoins. Chaque essai est réalisé en triple.

A la fin de la réaction dans les différentes conditions énoncées ci-dessus, la peroxydation lipidique est mesurée grâce à la détermination du pentane (nmole) résultant de la décomposition des peroxydes lipidiques. L'émission du pentane est directement proportionnelle à la peroxydation. Le pentane est mesuré par chromatographie en phase gazeuse : 0,1 ml de l'atmosphère du flacon où s'est produite la réaction sont injectés à l'aide d'une seringue Hamilton dans un chromatographe du type Varian 3600.

Ce chromatographe est équipé d'une colonne GF Moleculas Sieve Alltech (longueur: 1m ; ID : 1/8") à 140°C, le gaz vecteur utilisé étant l'azote (40 ml/min). L'étalonnage de la méthode est réalisé à l'aide de pentane pur.

Les résultats de ces mesures figurent dans les tableaux I et II ci-après.

**TABLEAU I**

| **Effet " anti-oxyferryl " (libération du pentane en nmole)** | | | | | | |
|---|---|---|---|---|---|---|
| **Concentration (µg/ml)** | **Chrysanthellum** | **Thé vert** | **Ginkgo biloba** | | **Tocophérol** | **Concentration (µM)** |
| **0,00** | **100,00** | **100,00** | **100,00** | | **100,00** | **0,00** |
| **0,15** | **101,39** | **110,83** | **122,77** | | **93,02** | **1,00** |
| **1,50** | **97,70** | **93,85** | **104,63** | | **82,17** | **2,50** |
| **15,00** | **82,77** | **87,61** | **93,00** | | **72,33** | **5,00** |
| **150,00** | **40,77** | **44,93** | **65,73** | | **57,07** | **7,50** |
| **300,00** | **11,17** | **24,33** | **35,47** | | **36,80** | **10,00** |

**TABLEAU II**

| **Effet " anti-hydroxyle " (libération du pentane)** | | | | | | |
|---|---|---|---|---|---|---|
| **Concentration (µg/ml)** | **Chrysanthellum** | **Thé vert** | **Ginkgo biloba** | | **Tocophérol** | **Concentration (µM)** |
| **0,00** | **100,00** | **100,00** | **100,00** | | **100,00** | **0,00** |
| **0,15** | **101,41** | **108,43** | **115,43** | | **119,67** | **1,00** |
| **1,50** | **86,67** | **97,30** | **100,37** | | **66,76** | **2,50** |
| **15,00** | **30,50** | **36,93** | **98,87** | | **13,70** | **5,00** |
| **150,00** | **12,73** | **23,03** | **53,87** | | **7,67** | **7,50** |
| **300,00** | **7,97** | **12,10** | **29,90** | | **5,59** | **10,00** |

Comme il ressort de ces résultats, les trois extraits végétaux inhibent la libération de pentane induite par le radical oxyferryl.

L'extrait de chrysanthellum indicum s'avère le meilleur inhibiteur de la peroxydation induite par le radical oxyferryl. L'extrait de thé vert présente un effet proche de celui du chrysanthellum alors que l'extrait de Ginkgo semble nettement moins protecteur.

Un effet protecteur des trois extraits est aussi observé sur la production de pentane induite par le radical hydroxyle. Cette protection est plus nette que celle observée pour l'induction induite par le radical oxyferryl.

Le tocophérol exerce aussi un effet protecteur. Celui-ci est nettement plus net sur la peroxydation induite par le radical OH° que celle induite par le radical peroxyferryl. Cette dissociation des effets semble plus importante que celle enregistrée avec les trois extraits végétaux.

La recherche d'un effet synergique entre l'extrait de chrysanthellum et l'extrait de thé vert, l'extrait de Ginkgo biloba ou le tocophérol a conduit à répéter les études précédentes mais avec des compositions incluant chacune un extrait de chrysanthellum et l'un de ces composés.

Les résultats de ces mesures figurent dans les tableaux III et IV ci-après.

**TABLEAU III**

| **Effet " anti-oxyferryl " du chrysanthellum en association (libération du pentane en nmole)** | | | | | | | |
|---|---|---|---|---|---|---|---|
| **Concentration (µg/ml) chrysanthellum** | **⊕ thé vert 1,5 µg/ml** | **⊕ thé vert 15 µg/ml** | **⊕ Ginkgo 15 µg/ml** | **⊕ Ginkgo 150 µg/ml** | | **⊕ Tocophérol 5 µM** | **⊕ Tocophérol 7,5 µM** |
| **0,00** | **100,00** | **100,00** | **100,00** | **100,00** | | **100,00** | **100,00** |
| **0,15** | **92,50** | **89,60** | **102,81** | **79,00** | | **101,53** | **94,67** |
| **1,50** | **84,77** | **58,50** | **95,77** | **71,90** | | **92,62** | **85,03** |
| **15,00** | **78,03** | **55,47** | **80,80** | **61,00** | | **81,28** | **76,00** |
| **150,00** | **41,63** | **31,60** | **42,67** | **34,10** | | **41,87** | **43,99** |
| **300,00** | **10,97** | **6,17** | **10,80** | **8,57** | | **11,90** | **9,33** |

**TABLEAU IV**

| **Effet " anti-hydroxyle " du chrysanthellum en association (libération du pentane en nmole)** | | | | | | | |
|---|---|---|---|---|---|---|---|
| **Concentration (µg/ml) chrysanthellum** | **⊕ thé vert 1,5 µg/ml** | **⊕ thé vert 15 µg/ml** | **⊕ Ginkgo 15 µg/ml** | **⊕ Ginkgo 150 µg/ml** | | **⊕ Tocophérol 5 µM** | **⊕ Tocophérol 7,5 µM** |
| **0,00** | **100,00** | **100,00** | **100,00** | **100,00** | | **100,00** | **100,00** |
| **0,15** | **96,53** | **85,50** | **101,80** | **87,07** | | **96,50** | **91,57** |
| **1,50** | **78,53** | **69,80** | **86,13** | **72,30** | | **79,64** | **78,67** |
| **15,00** | **26,43** | **22,57** | **28,07** | **30,40** | | **28,33** | **19,54** |
| **150,00** | **12,15** | **8,28** | **14,08** | **14,02** | | **12,80** | **8,83** |
| **300,00** | **4,34** | **4,76** | **6,43** | **6,38** | | **6,37** | **5,00** |

L'analyse des résultats est effectuée à partir des Tableaux V et VI ci-après pour lesquels les règles suivantes sont appliquées :
Protection = (100 - libération de pentane)
Protection totale = 100
Protection nulle = 0
Effet synergique si protection (A + B) > protection A + protection B
Effet additif total si protection (A + B) = protection A + protection B
Effet additif partiel si protection (A + B) < protection A + protection B avec protection (A + B) > protection A et protection (A + B) > protection B

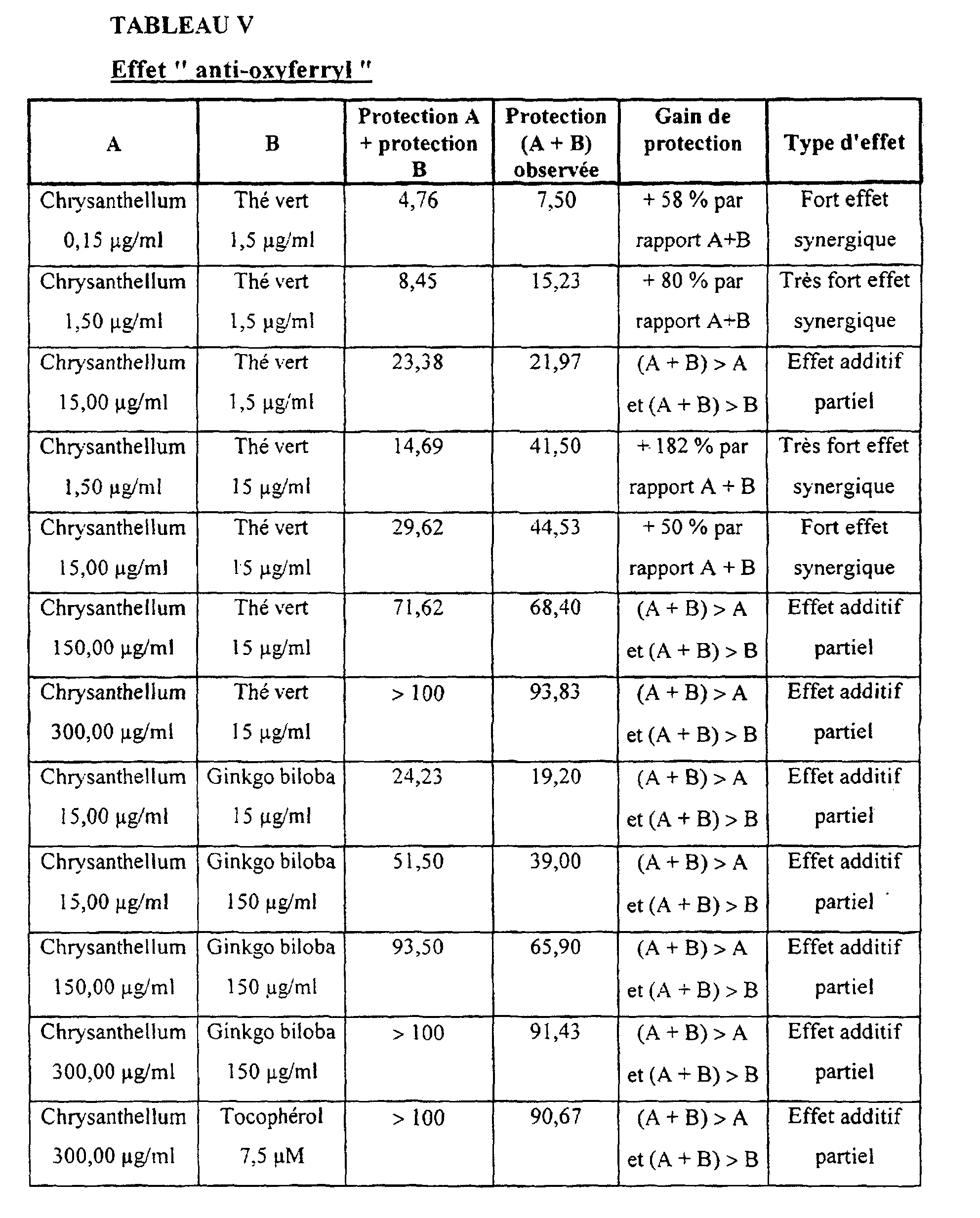

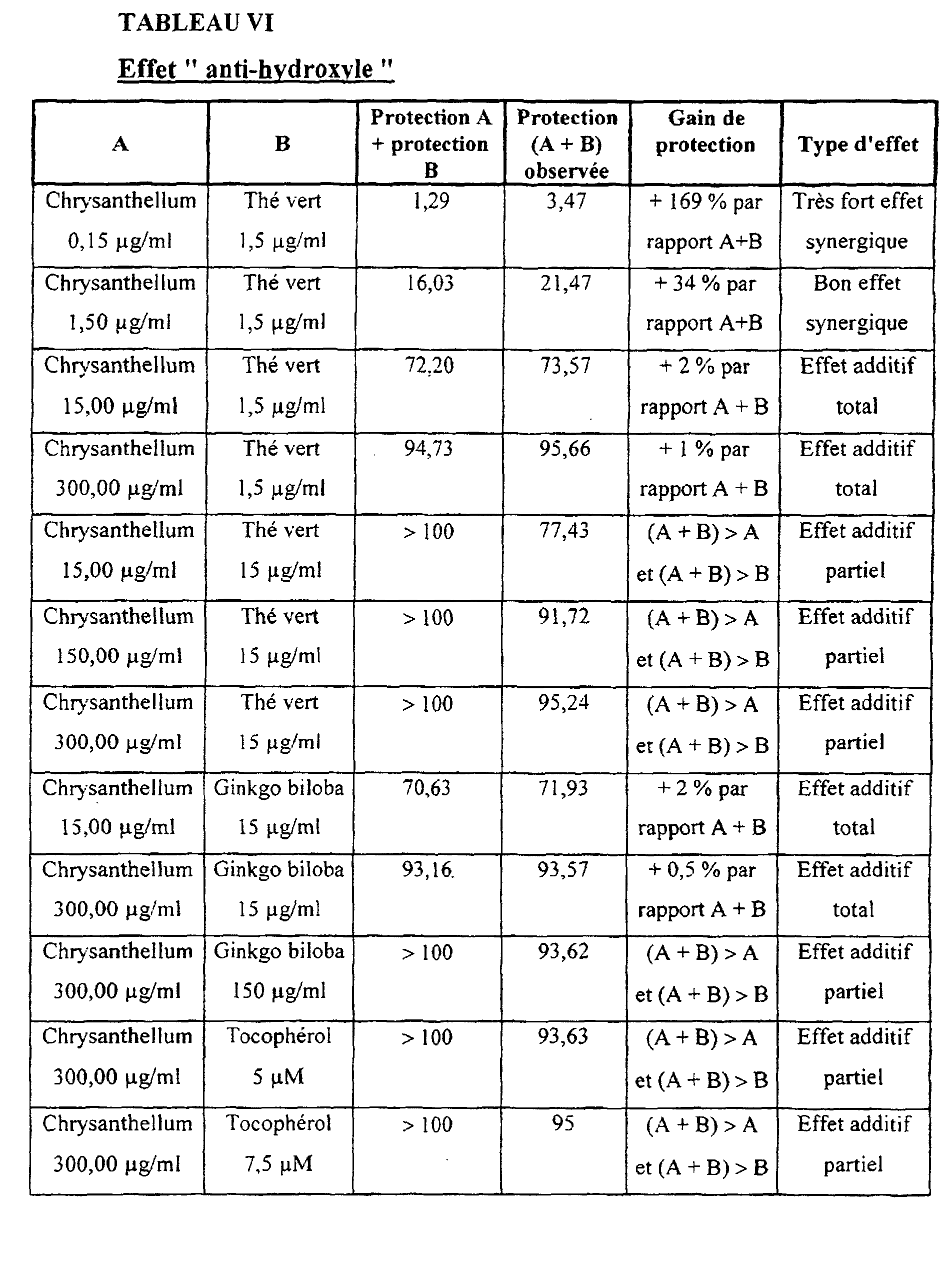

L'analyse des résultats de cette étude réalisée in vitro montre que contre toute attente, l'activité antiradicalaire excellente du chrysanthellum indicum (démontrée par le passé), peut être encore améliorée par addition d'autres molécules ou extraits végétaux connus pour leur propriétés antioxydantes et/ou antiradicalaires. Dans les différents cas étudiés (une étude exhaustive de toutes les associations possibles est difficilement réalisable), de très bons résultats ont été obtenus, quel que soit le modèle de production de radicaux libres utilisé.

Deux types d'effets positifs ont été constatés :
- Effet synergique (gain de protection observé allant de + 34 % à + 182 % par rapport à un effet additif). Cet effet synergique est observé dans plusieurs cas de figure (différentes concentrations en chrysanthellum et en extraits associés) et est particulièrement spectaculaire dans le cas d'une association avec le Thé vert. De tels effets synergiques sont remarquables.
- Effet additif total ou partiel. Compte tenu des très bons résultats obtenus avec le chrysanthellum seul, et compte tenu de l'existence d'un seuil maximal d'efficacité, il n'était pas évident que l'on puisse parvenir à obtenir des effets additifs. Des effets additifs totaux ou partiels ont pourtant été obtenus dans diverses associations chrysanthellum / autre substance antiradicalaire, ce qui est là encore assez remarquable.

La découverte de ces effets antiradicalaires synergiques est particulièrement intéressante notamment dans le domaine du soin cosmétique ou dermo-cosmétique destiné à :
. prévenir le vieillissement cutané et réparer les méfaits des agressions radicalaires sur la peau,
. protéger et réparer les cheveux exposés aux méfaits des rayonnements ultraviolets et d'autres facteurs environnementaux agressifs, générateurs d'un excès de radicaux libres.

Compte tenu de ces résultats, l'invention propose une composition cosmétique antiradicalaire utilisable pour les soins de la peau et des cheveux, et comprenant, d'une part, un extrait de chrysanthellum, et au moins l'un des composés repris dans la revendication 1.

Le chrysanthellum pourra consister en le chrysanthellum indicum, le chrysanthellum americanum ou le chrysanthellum procumbens.

Les composés phénoliques pourront comprendre des polyphénols, en particulier des flavonoïdes, ou des acides phénoliques.

Les extraits végétaux pourront être des extraits de Thé vert (Camelia sinensis), de Ginkgo biloba, de camomille, d'algues brunes du genre Ascophyllum. Ils pourront également consister en des extraits de plantes de la famille des Labiées, connues pour leurs propriétés antioxydantes (romarin, sauge, thym).

Les huiles végétales riches en tocophérols pourront consister en des huiles de germe de blé, de soja ou de sarrasin.

L'antioxydant d'origine naturelle ou synthétique comprend de l'acide caféique, de l'acide gallique, de l'acide ascorbique, du palmitate d'ascorbyle, de l'acide cinnamique, de l'acide nordihydroguaïarétique, de l'acide urique, de l'hespérétine, de l'hépéridine, de la quercétine, de l'acide rosmarinique, du rosmanol, du carnosol, de l'acide carnosique, de la vitamine E (tocophérols), du butyl-hydroxy-anisol, du butyl-hydroxy-toluène, de l'ethoxyquine, de l'acide ferulique, de l'hydroquinone, du tertiobutyl hydroquinone, du parahydroxyanisol, du gallate de propyle ou autres dérivés de l'acide gallique, de l'acétate de tocophérol, du linoléate de tocophérol, et/ou autres esters de tocophérols.

Les systèmes enzymatiques pourront, quant à eux, comprendre une superoxydismutase.

L'extrait de chrysanthellum et les extraits végétaux potentiellement associés pourront être des extraits secs, des extraits fluides hydrosolubles, des extraits huileux, des extraits secs en solution (dans de l'eau, des glycols, de la glycérine). Ces extraits végétaux ou ces molécules purifiées d'origine naturelle ou synthétique pourront également être encapsulés.

Bien entendu, les compositions selon l'invention pourront se présenter sous la forme d'émulsions simples ou multiples (crèmes ou laits eau/huile ou huile/eau, émulsions triples, micro-émulsions, émulsions à cristaux liquides), de gels aqueux ou huileux, de lotions aqueuses, huileuses, hydroalcooliques ou biphasiques, de sticks, ou de poudres ou de tout système vectorisé (système "à libération contrôlée" ou systèmes à "libération modulée"). Elles seront utilisées par voie topique.

Des exemples de formulation de la composition selon l'invention seront décrits ci-après, à titre d'exemples non limitatifs :

### Crème de jour protectrice

| | |
|---|---|
| Aqua | QSP 100 % |
| Esters émollients | 10 à 15 % |
| Glyceryl stearate & PEG-100 stearate | 4 à 6 % |
| Butylène Glycol | 2 à 5 % |
| Huile de germes de Blé | 2 à 5 % |
| Alcool cetylique | 1 à 5 % |
| Dimethicone | 1 à 4 % |
| Polymethyl methacrylate | 0,5 à 2 % |
| Parfum | 0,3 à 1 % |
| Conservateur | 0,1 à 0,8 % |
| Carbomer | 0,05 à 0,5 % |
| Triethanolamine | 0,05 à 0,5 % |
| Butyl Hydroxy Toluene | 0,05 à 0,1 % |
| Chrysanthellum indicum (extrait sec) | 0,0001 à 0,1 % |

## Revendications

1. Composition cosmétique pour la prévention du vieillissement de la peau et des cheveux, cette composition exploitant des effets antiradicalaires synergiques entre un extrait de chrysanthellum et un antioxydant,
**caractérisée en ce que** ledit antioxydant comprend de l'acide caféique, de l'acide gallique, de l'acide ascorbique du palmitate d'ascorbyle, de l'acide cinnamique, de l'acide nordihydroguaïarétique, de l'acide urique, de l'hespérétine, de l'hépéridine, de la quercétine, de l'acide romarinique, du rosmanol, du carnosol, de l'acide carnosique, de la vitamine E (tocophérols), du butyl-hydroxy-anisol, du butyl-hydroxy-toluène, de l'éthoxyquine, de l'acide ferulique, de l'hydroquinone, du tertiobutyl hydroquinone, du parahydroxyanisol, du gallate de propyle et autres dérivés de l'acide gallique, des tocophérols, de l'acétate de tocophérol, du linoléate de tocophérol, et/ou autres esters de tocophérol.

2. Composition selon la revendication 1,
**caractérisée en ce que** le chrysanthellum est du chrysanthellum indicum, du chrysanthellum americanum ou du chrysanthellum procumbens.

3. Composition selon l'une des revendications 1 et 2,
**caractérisée en ce que** la concentration en chrysanthellum comporte de 0,0001 % à 0,1 % en poids d'équivalents d'extraits secs.

4. Composition selon l'une des revendications précédentes,
**caractérisée en ce que** l'extrait de chrysanthellum et/ou des extraits végétaux potentiellement associés sont des extraits secs, des extraits fluides hydrosolubles, des extraits huileux, des extraits secs en solution.

5. Composition selon l'une des revendications 1 à 3,
**caractérisée en ce que** l'extrait de chrysanthellum et/ou des extraits végétaux potentiellement associés et/ou des molécules antioxydantes ou enzymatiques purifiées d'origine naturelle ou synthétique sont encapsulés.

6. Composition selon l'une des revendications précédentes,
**caractérisée en ce que** lesdits extraits végétaux potentiellement associés comprennent un polyphénol tel qu'un flavonoïde, et/ou un acide phénolique.

7. Composition selon la revendication 6,
**caractérisée en ce que** lesdits extraits végétaux potentiellement associés comprennent des extraits de thé vert, de Ginkgo biloba, de camomille et/ou d'algue brune du genre ascophyllum.

8. Composition selon la revendication 6,
**caractérisée en ce que** lesdits extraits végétaux potentiellement associés consistent en un extrait de plante de la famille des Labiées, telles que le romarin, la sauge ou le thym.

9. Composition selon l'une des revendications précédentes,
**caractérisée en ce que** lesdits extraits végétaux potentiellement associés sont des huiles végétales riche en tocophérols extraites de germes de blé, de. soja et/ou de sarrasin.

10. Composition selon l'une des revendications précédentes,
**caractérisée en ce que** les molécules enzymatiques comprennent une superoxydismutase.

11. Shampooing réparateur antiradicalaire,
**caractérisé en ce qu'**il comprend :
| | |
|---|---|
| Aqua | QSP 100 % |
| Cocamidopropyl betaïne | 15 à 20 % |
| Alkyl éther sulfates | 10 à 15 % |
| Caprypyl/Capryl glucoside | 2 à 10 % |
| Cocamide DEA | 2 à 4 % |
| Glycérine | 1 à 5 % |
| PEG-120 Methyl Glucose Dioleate | 1 à 5 % |
| Parfum | 0,2 à 1 % |
| Conservateur | 0,05 à 0,8 % |
| EDTA | 0,05 à 0,1 % |
| Ginkgo biloba (extrait sec) | 0,0001 à 1 % |
| Chrysanthellum indicum (extrait sec) | 0,0001 à 0,1 % |

12. Masque capillaire,
**caractérisé en ce qu'**il comprend :
| | |
|---|---|
| Aqua | QSP 100 % |
| PEG-6 Stearate & Ceteth-20 & Glyceryl stearate & Steareth-20 | 5 à 10 % |
| Alcool cetylique | 2 à 5 % |
| Quatemium-80 | 1 à 5 % |
| Beurre de Karité | 1 à 5 % |
| Huiles minérales et esters émollients . | 1 à 5 % |
| Glycérine | 1 à 5 % |
| Dimethicone copolyol | 1 à 3 % |
| Parfum | 0,1 à 1 % |
| Conservateur | 0,1 à 0,7 % |
| Carbomer | 0,05 à 0,5 % |
| Triethanolamine | 0,05 à 0,5 % |
| Camelia sinensis = Thé vert (extrait sec) | 0,0001 à 1 % |
| Chrysanthellum indicum (extrait sec) | 0,0001 à 0,1 % |

13. Crème de jour protectrice,
**caractérisée en ce qu'**elle comprend :
| | |
|---|---|
| Aqua | QSP 100 % |
| Esters émollients | 10 à 15 % |
| Glyceryl stearate & PEG-100 stearate | 4 à 6 % |
| Butylène Glycol | 2 à 5 % |
| Huile de germes de Blé | 2 à 5 % |
| Alcool cetylique | 1 à 5 % |
| Dimethicone | 1 à 4 % |
| Polymethyl methacrylate | 0,5 à 2 % |
| Parfum | 0,3 à 1 % |
| Conservateur | 0,1 à 0,8 % |
| Carbomer | 0,05 à 0,5 % |
| Triethanolamine | 0,05 à 0,5 % |
| Butyl Hydroxy Toluene | 0,05 à 0,1 % |
| Chrysanthellum indicum (extrait sec) | 0,0001 à 0,1 % |

## Patentansprüche

1. Kosmetische Zusammensetzung zum Verhindern der Alterung von Haut und Haaren, wobei diese Zusammensetzung radikalhemmende, synergetische Wirkungen zwischen einem Chrysanthemen-Extrakt und einem Antioxydationsmittel nutzt,
**dadurch gekennzeichnet dass**, das Antioxydationsmittel umfasst: Kaffeesäure, Gallussäure, Ascorbylpalmitatascorbinsäure, Zimtsäure, Nordihydroguajaretsäure, Harnsäure, Hesperetin, Heperidin, Quercetin, Rosmarinsäure, Rosmanol, Carnosol, Carnosinsäure, Vitamin E (Tocopherole), Butylhydroxyanisol, Butylhydroxytoluol, Ethoxyquin, Ferulasäure, Hydrochinon, t-Butylhydrochinon, Parahydroxyanisol, Propylgallat und andere Derivate der Gallussäure, Tocopherole, Tocopherolacetat, Tocopherollinoleat und/oder andere Tocopherolester.

2. Zusammensetzung nach Anspruch 1, **dadurch gekennzeichnet, dass** es sich bei der Chrysanthemenart um Chrysanthemum indicum, Chrysanthemum americanum oder Chrysanthemum procumbens handelt.

3. Zusammensetzung nach einem der Ansprüche 1 und 2, **dadurch gekennzeichnet, dass** die Konzentration an Chrysantheme 0,0001 Gew.-% bis 0,1 Gew.-% an Trockenextrakt-Äquivalenten umfasst.

4. Zusammensetzung nach einem der vorhergehenden Ansprüche, **dadurch gekennzeichnet, dass** der Chrysanthemen-Extrakt und/oder potentiell assoziierte Pflanzenextrakte Trockenextrakte, wasserlösliche Fluidextrakte, ölige Extrakte und Trockenextrakte in Lösung sind.

5. Zusammensetzung nach einem der vorhergehenden Ansprüche 1 bis 3 **dadurch gekennzeichnet, dass** der Chrysanthemen-Extrakt und/oder die potentiell assoziierten Pflanzenextrakte und/oder die gereinigten oxidationshemmenden oder enzymatischen Moleküle natürlicher oder synthetischer Herkunft eingekapselt sind.

6. Zusammensetzung nach einem der vorhergehenden Ansprüche, **dadurch gekennzeichnet, dass** die besagten potentiell assoziierten Pflanzenextrakte ein Polyphenol, wie z.B. ein Flavonoid, und/oder eine Phenolsäure aufweisen.

7. Zusammensetzung nach Anspruch 6, **dadurch gekennzeichnet, dass** die besagten potentiell assoziierten Pflanzenextrakte Extrakte aus grünem Tee, Gingko biloba, Kamille und/oder Braunalgen der Gattung Ascophyllum aufweisen.

8. Zusammensetzung nach Anspruch 6, **dadurch gekennzeichnet, dass** die besagten potentiell assoziierten Pflanzenextrakte aus einem Pflanzenextrakt der Familie der Lippenblütler, wie z.B. Rosmarin, Salbei oder Thymian bestehen können.

9. Zusammensetzung nach einem der vorhergehenden Ansprüche, **dadurch gekennzeichnet, dass** die besagten potentiell assoziierten Pflanzenextrakte Pflanzenöle sind, die reich an Tocopherolen sind, extrahiert aus Weizen-, Soja- und/oder Buchweizenkeimen.

10. Zusammensetzung nach einem der vorhergehenden Ansprüche, **dadurch gekennzeichnet, dass** die enzymatischen Moleküle eine Superoxydismutase aufweisen.

11. Radikalhemmendes, aufbauendes Shampoo, **dadurch gekennzeichnet, dass** es folgendes umfasst:
| | |
|---|---|
| Aqua | Rest auf 100 % |
| Cocamidopropylbetain | 15 bis 20 % |
| Alkylethersulfate | 10 bis 15 % |
| Caprypyl/Caprylglucosid | 2 bis 10 % |
| Cocamid-DEA | 2 bis 4 % |
| Glycerin | 1 bis 5 % |
| PEG-120-Methylglucosedioleat | 1 bis 5 % |
| Parfum | 0,2 bis 1 % |
| Konservierungsmittel | 0,05 bis 0,8 % |
| EDTA | 0,05 bis 0,1 % |
| Gingko biloba (Trockenextrakt) | 0,0001 bis 1 % |
| Chrysanthemum indicum (Trockenextrakt) | 0,0001 bis 0,1 % |

12. Haarkur, **dadurch gekennzeichnet, dass** sie folgendes umfasst:
| | |
|---|---|
| Aqua | Rest auf 100 % |
| PEG-6-Stearat & Ceteth-20 & | |
| Glycerylstearat & Steareth-20 | 5 bis 10 % |
| Cetylalkohol | 2 bis 5 % |
| Quaternium-80 | 1 bis 5 % |
| Karitébutter . | 1 bis 5 % |
| Mineralöle und weichmachende Ester | 1 bis 5 % |
| Glycerin | 1 bis 5 % |
| Dimethiconcopolyol | 1 bis 3 % |
| Parfum | 0,1 bis 1 % |
| Konservierungsmittel | 0,1 bis 0,7 % |
| Carbomer | 0,05 bis 0,5 % |
| Triethanolamin . | 0,05 bis 0,5 % |
| Camelia sinensis = grüner Tee (Trockenextrakt) | 0,0001 bis 1 % |
| Chrysanthemum indicum (Trockenextrakt) | 0,0001 bis 0,1 % |

13. Schützende Tagescreme, **dadurch gekennzeichnet, dass** sie folgendes umfasst:
| | |
|---|---|
| Aqua | Rest auf 100 % |
| Weichmachende Ester | 10 bis 15 % |
| Glycerylstearat & PEG-100-Stearat | 4 bis 6 % |
| Butylenglykol | 2 bis 5 % |
| Weizenkeimöl | 2 bis 5 % |
| Cetylalkohol | 1 bis 5 % |
| Dimethicon | 1 bis 4 % |
| Polymethylmethacrylat | 0,5 bis 2 % |
| Parfum | 0,3 bis 1 % |
| Konservierungsmittel | 0,1 bis 0,8 % |
| Carbomer | 0,05 bis 0,5 % |
| Triethanolamin | 0,05 bis 0,5 % |
| Butylhydroxytoluol | 0,05 bis 0,1 % |
| Chrysanthemum indicum (Trockenextrakt) | 0,0001 bis 0,1 % |

## Claims

1. Cosmetic composition for the prevention of skin and hair aging and based on the synergistic anti-radical effects of a chrysanthellum extract and an antioxidant,
wherein said antioxidant includes cafeic acid, gallic acid, ascorbyl palmitate ascorbic acid, cinnamic acid, nordihydroguaiaretic acid, uric acid, hesperitine, heperidine, quercetin, romarinic acid, rosmanol, carnosol, camosic acid, vitamin E (tocopherols), butyl-hydroxy-anisol, butyl-hydroxy-toluene, ethoxyquine, ferulic acid, hydroquinone, hydroquinone tertiobutyl, parahydroxyanisol, propyl gallate and other derivatives of gallic acid, tocopherols, tocopherol acetate, tocopherol linoleate and/or other tocopherol esters.

2. Composition according to claim 1, wherein the chrysanthellum is Chrysanthellum indicum, Chrysanthellum americanum or Chrysanthellum procumbens.

3. Composition according to claims 1 and 2, wherein the concentration of Chrysanthellum indicum comprises from 0.0001 % to 0.1 % by weight of equivalents of dry extracts.

4. Composition according to one of the preceding claims, wherein the extract of Chrysanthellum indicum and/or the plant extracts possibly combined are dry extracts, water-soluble fluid extracts, oily extracts or dry extracts in solution.

5. Composition according to one of claim 1 to 3, wherein the extract of Chrysanthellum indicum and/or the plant extracts possibly combined and/or the antioxidant or purified enzymatic molecules of natural or synthetic origin are encapsulated.

6. Composition according one of the preceding claims, wherein the plant extract which is rich in phenolic compounds comprises a polyphenol such as a flavonoid and/or a phenolic acid.

7. Composition according to claim 6, wherein the plant extract comprises extracts of green tea, of Ginkgo biloba, of camomile and/or of brown alga of the genus ascophyllum.

8. Composition according to claim 6, wherein the plant extract may consist of an extract of a plant from the Labiatae family, such as rosemary, sage or thyme.

9. Composition according to one of the preceding claims, wherein the plant oil which is rich in tocopherols is extracted from wheatgerm, from soybean and/or from buckwheat.

10. Composition according to one of the preceding claims, wherein the enzymatic systems comprise a superoxide dismutase.

11. Radical-scavenging repairing shampoo, comprising:
| | |
|---|---|
| Water | QS 100% |
| Cocamidopropyl betaine | 15 to 20% |
| Alkyl ether sulphates | 10 to 15% |
| Caprylyl/capryl glucoside | 2 to 10% |
| Cocamide DEA | 2 to 4% |
| Glycerol | 1 to 5% |
| PEG-120 methyl glucose dioleate | 1 to 5% |
| Fragrance | 0.2 to 1% |
| Preserving agent | 0.05 to 0.8% |
| EDTA | 0.05 to 0.1% |
| Ginkgo biloba (dry extract) | 0.0001 to 1% |
| Chrysanthellum indicum (dry extract) | 0.0001 to 0.1% |

12. Hair mask, comprising:
| | |
|---|---|
| Water | QS 100% |
| PEG-6 Stearate & Ceteth-20 & glyceryl stearate & | 5 to 10% |
| Steareth-20 | |
| Cetyl alcohol | 2 to 5% |
| Quatemium-80 | 1 to 5% |
| Karite butter | 1 to 5% |
| Mineral oils and emollient esters | 1 to 5% |
| Glycerol | 1 to 5% |
| Dimethicone copolyol | 1 to 3% |
| Fragrance | 0.1 to 1% |
| Preserving agent | 0.1 to 0.7% |
| Carbomer | 0.05 to 0.5% |
| Triethanolamine | 0.05 to 0.5% |
| Camelia sinensis = Green tea (dry extract) | 0.0001 to 1% |
| Chrysanthellum indicum (dry extract) | 0.0001 to 0.1% |

13. Protective day cream, comprising:
| | |
|---|---|
| Water | QS 100% |
| Emollient esters | 10 to 15% |
| Glyceryl stearate & PEG-100 stearate | 4 to 6% |
| Butylene glycol | 2 to 5% |
| Wheatgerm oil | 2 to 5% |
| Cetyl alcohol | 1 to 5% |
| Dimethicone | 1 to 4% |
| Polymethyl methacrylate | 0.5 to 2% |
| Fragrance | 0.3 to 1% |
| Preserving agent | 0.1 to 0.8% |
| Carbomer | 0.05 to 0.5% |
| Triethanolamine | 0.05 to 0.5% |
| Butylated hydroxytoluene | 0.05 to 0.1% |
| Chrysanthellum indicum (dry extract) | 0.0001 to 0.1% |
